## Europäisches Patentamt

## European Patent Office

## Office européen des brevets

(11) Veröffentlichungsnummer: **0 324 915**
A1

# EUROPÄISCHE PATENTANMELDUNG

(21) Anmeldenummer: **88119166.2**

(51) Int. Cl.⁴: **B01D 13/00 , C07C 29/76**

(22) Anmeldetag: **18.11.88**

(30) Priorität: **14.01.88 DE 3800791**

(43) Veröffentlichungstag der Anmeldung:
**26.07.89 Patentblatt 89/30**

(84) Benannte Vertragsstaaten:
**CH DE FR GB IT LI NL**

(71) Anmelder: **HÜLS AKTIENGESELLSCHAFT**
**- RSP Patente / PB 15 - Postfach 13 20**
**D-4370 Marl 1(DE)**

(72) Erfinder: **Sridhar, Srinivasan, Dr.**
**Lembecker Pfad 52**
**D-4370 Marl(DE)**

(54) **Verfahren zum Abtrennen von Wertstoffen aus wässrigen Lösungen.**

(57) Wertstoffe lassen sich durch Extrahieren und/oder Destillieren aus wäßrigen Lösungen abtrennen; die Lösung kann vorher durch Umkehrosmose aufkonzentriert werden.

Die Wertstoffe werden allein durch Destillieren nach dem Aufkonzentrieren der Lösung durch gegebenenfalls mehrstufige Umkehrosmose abgetrennt. Der Wertstoff verläßt die Destillationskolonne mit dem Sumpfstrom oder dem Kopfstrom. Bei einem Alkohol als Wertstoff liegt der Druck der Umkehrosmose bei 1 bis 10 MPa und die Temperatur bei 5 bis 60 °C.

Das Verfahren wird verwendet speziell zum Abtrennen von aliphatischen Alkoholen mit 2 bis 4 C-Atomen (z. B. Butandiol-2,3) aus einer Fermenterbrühe.

Fig. 1

EP 0 324 915 A1

## Verfahren zum Abtrennen von Wertstoffen aus wäßrigen Lösungen

Die Erfindung betrifft ein Verfahren zum Abtrennen von Wertstoffen wie niedermolekularen Alkoholen, insbesondere Butandiol-2,3 , aus deren wäßrigen Lösungen, die beispielsweise bei einer fermentativen Herstellung entstanden sind. Die Erfindung bezweckt, diese Abtrennung wirtschaftlicher zu gestalten.

Die Isolierung des z. B. bei einer Fermentation in kleinen Konzentrationen (5 - 10 Gew.-%) anfallenden Alkohols aus der Fermenterbrühe ist aufwendig und umständlich. Die Alkohole sind mit Wasser in allen Verhältnissen mischbar. Butandiol siedet bei einer wesentlich höheren Temperatur (180 °C/100 kPa) als Wasser. Eine destillative Abtrennung erfordert in diesem Fall die unwirtschaftliche Verdampfung des gesamten Wassers. Der Einsatz von Wasserdampf zur Gewinnung von Butandiol wurde beschrieben; dabei entsteht zwar eine reine, aber verdünnte Lösung, die wieder destillativ entwässert werden muß (Ind. Eng. Chem. 38 (1946), S. 913). Ein weiterer umständlicher Weg ist die chemische Umsetzung des Diols zu Formal mittels Formaldehyd und die Umsetzung des Formals in Butandiol mittels Methanol (Ind. Eng. Chem. 37 (1945), S. 865).

Als Alternative zur Destillation bietet sich eine Extraktion an. Als Extraktionsmittel eignen sich beispielsweise Fettalkohole, wobei die niedermolekularen Alkohole, darunter insbesondere n-Butanol, ein hohes Aufnahmevermögen für das Diol haben (Ind. Eng. Chem. 37 (1945), S. 890). n-Butanol ist aber zum Teil löslich in Wasser und verbleibt im Raffinat, was eine zusätzliche Aufarbeitung erfordert. Daher wurden auch als Alternativen weniger lösliche Ester erwähnt, die aber ein entscheidend geringeres Lösevermögen für das Diol haben. Neuere Bestrebungen, insbesondere zur Isolierung von Ethanol aus Fermenterbrühen, zielen auf mehrfache Extraktion mit Lösemitteln, z. B. n-Hexanol und n-Dodekan, oder auf die Bereitstellung hochmolekularer, im Wasser wenig löslicher Extraktionsmittel, die aber wie die Fettalkohole auch Wasser aufnehmen. Es werden Polyoxaalkohole, Polyoxa alkanole und Polyoxaalkanpolyole erwähnt (DE-OS 31 12 603). Auch diese Lösemittel weisen kein mit Butanol und Hexanol vergleichbares Aufnahmevermögen auf, wie bereits am Beispiel von Ethanol als Wertstoff nachgewiesen wurde. Die starke Emulgatorwirkung solcher hochmolekularer Mittel sowie geringe Dichteunterschiede zwischen der organischen und wäßrigen Phase (insbesondere bei Butandiol) erschweren die Extraktion erheblich. Das Lösemittel im Extrakt kann durch Destillation oder Umkehrosmose vom gelösten Wasser und Wertstoff befreit werden, aber dabei verbleiben im Lösemittel auch unerwünschte systemeigene Hochsieder wie das Dimere von Acetoin im Falle von Butandiol. Der erforderliche Extraktionsaufwand ist größer als bei Butanol bzw. Hexanol, und das Raffinat enthält noch Alkoholreste und Spuren von Lösemitteln, die zu Verlusten und Entsorgungsproblemen führen. Auch der Verfügbarkeit und den Herstellkosten solcher Lösemittel muß Rechnung getragen werden.

Nach dem Stand der Technik zielen alle Bestrebungen darauf, unter Verzicht auf das gute Aufnahmevermögen eines verfügbaren niedermolekularen Extraktionsmittels Verbindungen höheren Molekulargewichtes zur Extraktion heranzuziehen, die weniger wasserlöslich sind.

Gemäß DE-OS 36 23 827 kann man das Extraktionsverfahren verbessern, indem man dem Extraktionsschritt einen Umkehrosmoseschritt vorschaltet. Mittels der Umkehrosmose wird die wäßrige Lösung des Alkohols aufkonzentriert; das Wasser verläßt die Umkehrosmosestufe als Permeat. Gleichzeitig wird das Extraktionsmittel aus dem Retentat der Extraktionsstufe in den Extraktionskreislauf in wasserarmer Form zurückgeführt. Wegen des Einsatzes eines Extraktionsmittels läßt sich der Alkohol aus dem Extrakt in gewissen Fällen destillativ nur schwer abtrennen; der abzutrennende Alkohol soll nicht nur wasserarm oder wasserfrei sein, sondern auch frei von Extraktionsmittel. Das führt zu einer relativ aufwendigen Trennung des Extraktionsmittels vom Alkohol.

Aufgabe der Erfindung ist, das Verfahren zum Abtrennen von Wertstoffen aus wäßrigen Lösungen auch für solche Lösungen zu finden, bei denen das Abtrennen des Wertstoffs bei Verwendung eines Extraktionsmitteis schwierig wird.

Diese Aufgabe wird erfindungsgemäß gelöst durch ein Verfahren mit folgenden kennzeichnenden Merkmalen:
- Aufkonzentrieren einer wäßrigen Lösung des Wertstoffes durch Umkehrosmose und Ausschleusen eines Teils des Wassers als Permeat der Umkehrosmose,
- Destillieren des aufkonzentrierten Retentats der Umkehrosmose und Ausschleusen des wasserfreien Wertstoffes oder seines wäßrigen Azeotrops aus der Destillationskolonne mit dem Sumpfstrom oder Kopfstrom.

Die wäßrige Lösung des Wertstoffs kann auch durch eine mehrstufige Umkehrosmose aufkonzentriert werden, wobei das Retentat einer Stufe in den Zulaufstrom der vorhergehenden Stufe zurückgeführt wird.

Für Lösungen mit niedermolekularen Alkoholen als Wertstoff wird die Umkehrosmose bei einem Druck von 1 bis 10, vorzugsweise 4 bis 6 MPa und einer Temperatur von 5 bis 60, vorzugsweise 20 bis 40 °C durchgeführt.

Als wäßrige Lösung des Wertstoffes kann auch eine filtrierte Fermenterbrühe eingesetzt werden, die den Wertstoff - in der Regel in verdünnter Form - enthält. Bei den Wertstoffen handelt es sich bevorzugt um aliphatische Alkohole mit 2 bis 4 C-Atomen, speziell auch um Butandiol-2,3.

Das erfindungsgemäße Verfahren hat folgende Vorteile:

- Das Aufkonzentrieren der Lösung durch Umkehrosmose wird beibehalten; die in gewissen Fällen mit der Extraktion des Wertstoffes verbundenen Schwierigkeiten treten jedoch nicht mehr auf.

- Die destillative Trennung des Retentats der Umkehrosmose in Wasser und Wertstoff ist ein relativ einfacher Vorgang. Er ist besonders wirtschaftlich in den Fällen, in denen die Konzentration des Wertstoffs in der Lösung bereits hoch ist oder durch Umkehrosmose auf hohe Werte im Retentat gebracht wird, der Wertstoff einen wesentlich höheren Siedepunkt als Wasser hat und mit Wasser kein Azeotrop bildet.

- Der Umkehrosmosestufe wird kein Rückstrom aus einer nachgeschalteten Stufe, z. B. einer Extraktionsstufe, zugeführt.

- Das Verfahren ist nicht nur bei Lösungen von aliphatischen Alkoholen anwendbar, sondern auch bei anderen wasserlöslichen Stoffen, bei denen das Extraktionsverfahren schwierig ist oder nicht angewendet werden kann, sofern für die Umkehrosmose eine geeignete Membran verfügbar ist, die den abzutrennenden wasserlöslichen Stoff zurückhält.

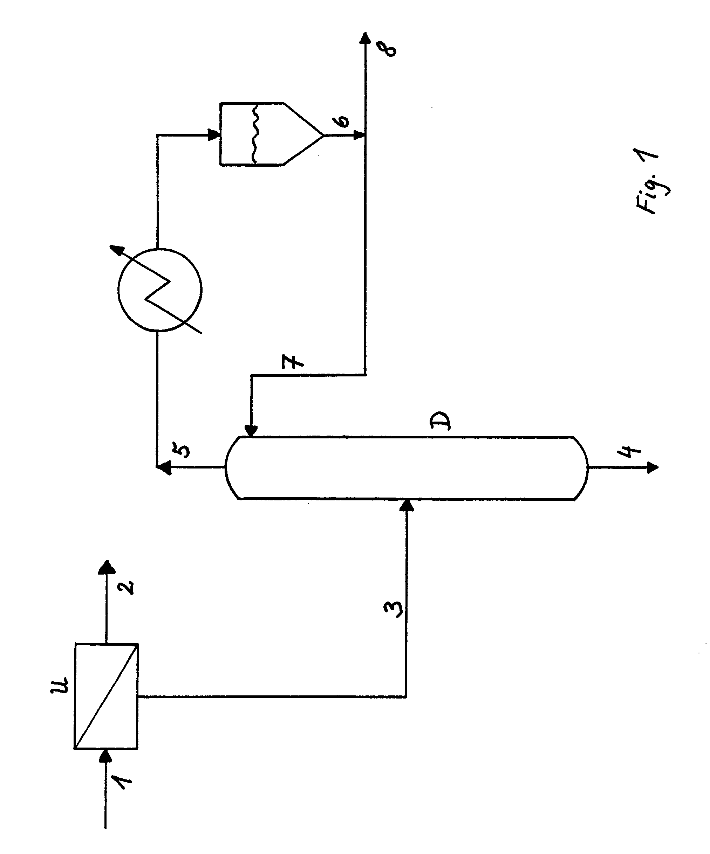

Weitere Einzelheiten zeigt die Figur 1. Die Umkehrosmosestufe (U) wird über den Strom (1) mit der wäßrigen Lösung des Wertstoffes beschickt; der Permeatstrom (2) besteht aus Wasser, das Wertstoff-frei oder Wertstoff-arm ist. Der Wertstoff-haltige wäßrige Retentatstrom (3) wird der Destillationsstufe (D) zugeführt und dort in einen Wertstoff-haltigen Strom und einen wäßrigen Strom getrennt. Je nach dem Verhältnis der Siedepunkte von Wertstoff und Wasser kann der Wertstoff in den Sumpfstrom oder den Kopfstrom gehen. Der Sumpfstrom (Wasser oder Wertstoff) verläßt die Destillationskolonne als Strom (4), der Kopfstrom (Wertstoff oder Wasser) als Strom (5). Der Kopfstrom wird kondensiert. Der Kondensatstrom (6) wird teilweise als Strom (7) zum Kopf der Kolonne zurückgeführt und teilweise als Strom (8) entnommen.

Das Verfahren wird an Hand der folgenden Beispiele weiter erläutert, ohne darauf beschränkt zu sein. Für die Beispiele wurden Lösungen verwendet, die Butandiol-2,3 als Wertstoff sowie weitere Substanzen in geringerer Konzentration enthielten, und deren Zusammensetzung in Tabelle 1 angegeben ist. Die "synthetische" Lösung wurde durch Mischen der angegebenen Stoffe hergestellt. Die Fermenterbrühe wurde einem Reaktor zur biotechnischen Herstellung von Butandiol-2,3 entnommen. Alle Lösungen wurden zunächst über ein Filter mit 0,2 Mikrometer Porenweite mikrofiltriert.

In den Tabellen sind Konzentrationen und Anteile stets in Massenprozent angegeben.

Beispiel 1: Aufkonzentrieren durch Umkehrosmose

Für die Umkehrosmose wurde ein Membranstapel mit 0,36 m² Membranfläche und der Membran HR 98 oder HR 99 (Filmtech) eingesetzt.

In den Versuchen 1 und 2 wurden zwei Teilmengen der synthetischen Lösung aufkonzentriet. Die Konzentration von Butandiol-2,3 stieg von 6 % auf ca. 10 % im Retentat. Die Retentate aus beiden Versuchen wurden vereinigt und im Versuch 3 weiter auf ca. 15 % aufkonzentriert; das Retentat aus Versuch 3 wurde also auf das 2,5-fache der für die Versuche 1 und 2 eingesetzten Lösung aufkonzentriert. Jeder Versuch dauerte 2 bis 4 Stunden.

Der Verlust an Butandiol-2,3 betrug bei allen drei Versuchen weniger als 1 % der in der vorgelegten Lösung vorhandenen Menge. Der Essigsäure-Schlupf betrug 2 bis 4 % der eingesetzten Essigsäuremenge.

In den Versuchen 4 und 5 wurde Fermenterbrühe auf knapp 10 % Butandiol-2,3 im Retentat aufkonzentriert. Das Aufkonzentrierungsverhältnis betrug in beiden Fällen über 2. Etwa 2 % der eingesetzten Menge an Butandiol-2,3 gingen verloren. Ein wesentlicher Unterschied zwischen den beiden Membranentypen ist nicht zu erkennen. Weitere Einzelheiten enthält Tabelle 2.

Beispiel 2: Destillation des Retentats der Umkehrosmose

Zur Reingewinnung von Butandiol-2,3 wurde das Retentat der Umkehrosmose aus Versuch 3 zunächst in einer Füllkörperkolonne (Nennweite 40 mm, Füllkörperhöhe 1 m, gefüllt mit 4 X 4-Raschigringen) bei 26 kPa fraktioniert. Das Rücklaufverhältnis betrug etwa 0,4, die Kopftemperatur etwa 57 °C. Das Destillat umfaßt 76 % der vorgelegten Retentat-Menge; es bestand zu 99,7 % aus Wasser und enthielt den größten Teil der mit Wasser übergehenden weiteren Stoffe.

Der Rückstand (ca. 24 % der vorgelegten Menge) wurde in einer Spaltrohrkolonne (Nennweite 40 mm) bei 1,3 kPa weiter fraktioniert. Das Rücklaufverhältnis betrug etwa 4, die Kopftemperatur etwa 59 °C. Dabei wurde Butandiol-2,3 in einer Reinheit von über 99 % erhalten.

Tabelle 1

| Zusammensetzung der Lösung | | | |
|---|---|---|---|
| | Synthetische Lösung | Fermenterbrühe | |
| eingesetzt im Versuch | 1 bis 3 | 4 | 5 |
| Butandiol-2,3 | 6,0 | 4,52 | 4,30 | % |
| Diacetyl | 0,1 | - | - | % |
| Ethanol | 1,0 | 0,67 | 0,65 | % |
| Milchsäure | 0,5 | - | - | % |
| Essigsäure | 0,2 | 2,0 | 1,9 | % |
| Melasse | 2,0 | - | - | % |
| Acetoin | - | 0,038 | 0,017 | % |
| Wasser | 90,2 | 92,8 | 93,1 | % |

Tabelle 2

| Aufkonzentrieren durch Umkehrosmose | | | | | | |
|---|---|---|---|---|---|---|
| Versuch | 1 | 2 | 3 | 4 | 5 | |
| Membran | HR 98 | | | | HR 99 | |
| Druck | 5 | 5 | 7,5 | 7 | 7 | MPa |
| Permeatrate | 7,8 | 7,8 | 6,5 | 6,8 | 10,1 | $l/m^2 \cdot h$ |
| Einsatzlösung | Synthetische Lösung | | Retentate aus Beispiel 1 und 2 | Fermenterbrühe | | |
| Einsatzmenge | 9922 | 8116 | 9099 | 8085 | 7342 | g |
| Permeatmenge | 3632 | 3595 | 2948 | 4403 | 3865 | g |
| Retentatmenge | 6290 | 4521 | 6151 | 3682 | 3477 | g |
| Aufkonzentrierungsverhältnis (Einsatz/Retentat) | 1,58 | 1,80 | 1,48 | 2,20 | 2,11 | |
| Butandiol-Konzentration im Retentat | 9,4 | 12,0 | 15,4 | 9,7 | 9,1 | % |
| Verlust an Butandiol bezogen auf eingesetzte Menge | 0,36 | 0,47 | 0,87 | 2,0 | 1,5 | % |

**Ansprüche**

1. Verfahren zum Abtennen von Wertstoffen aus einer wäßrigen Lösung, gekennzeichnet durch
- Aufkonzentrieren einer wäßrigen Lösung des Wertstoffs durch Umkehrosmose und Ausschleusen eines Teils des Wassers als Permeat der Umkehrosmose,
- Destillieren des aufkonzentrierten Retentats der Umkehrosmose und Ausschleusen des wasserfreien Wertstoffs oder seines wäßrigen Azeotrops aus der Destillationskolonne mit dem Sumpfstrom oder dem Kopfstrom.

2. Verfahren nach Anspruch 1, gekennzeichnet durch
- Aufkonzentrieren einer wäßrigen Lösung des Wertstoffs durch mehrstufige Umkehrosmose.

3. Verfahren nach den Ansprüchen 1 und 2, gekennzeichnet durch
- Aufkonzentrieren einer Fermenterbrühe als wäßriger Lösung des Wertstoffs.

4. Verfahren nach den Ansprüchen 1 bis 3, gekennzeichnet durch - Aufkonzentrieren einer wäßrigen Lösung von niedermolekularen Alkoholen als Wertstoff durch Umkehrosmose bei einem Druck von 1 bis 10, vorzugsweise 4 bis 6 MPa und einer Temperatur von 5 bis 60, vorzugsweise 20 bis 40 $^\circ$C.

5. Verfahren nach den Ansprüchen 1 bis 4, gekennzeichnet durch
- Aufkonzentrieren einer wäßrigen Lösung von aliphatischen Alkoholen mit 2 bis 4 C-Atomen als Wertstoff.

6. Verfahren nach den Ansprüchen 1 bis 5, gekennzeichnet durch
- Aufkonzentrieren einer wäßrigen Lösung von Butandiol-2,3 als Wertstoff.

Fig. 1

EP 0 324 915 A1

0.Z. 4291

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | JOURNAL OF MEMBRANE SCIENCE Band 12, Nr. 1, November 1982, Seiten 1-26, Elsevier Scientific Publishing Company, Amsterdam, NL; G.D. MEHTA:"Comparison of membrane processes with distillation for alcohol/water separation." * Seite 1, Zusammenfassung; Seite 13, Zeilen 42-45; Seite 14, Zeilen 1-4; Figur 2 * | 1-3,5 | B 01 D 13/00 C 07 C 29/76 |
| P,X | EP-A-0 253 091 (HUELS AG) * Ansprüche 9,10; Zusammenfassung; Figur 1 * | 4-6 | |
| A | EP-A-0 240 803 (GKSS FORSCHUNGSZENTRUM GEESTHACHT) * Anspruch 1 * | 1,3,5 | |
| A | DE-A-3 037 736 (G. TUSEL et al.) * Anspruch 1 * | 1 | |
| A | PATENT ABSTRACTS OF JAPAN Band 7, Nr. 139 (C-171)(1284), 17. Juni 1983; & JP - A - 58 55001 (KURARAY K.K.) 01.04.1983 * Zusammenfassung * | | **RECHERCHIERTE SACHGEBIETE (Int. Cl.4)** B 01 D 13/00 B 01 D 3/36 C 07 C 29/76 C 07 C 29/82 C 07 C 29/86 |

**Der vorliegende Recherchenbericht wurde für alle Patentansprüche erstellt**

| Recherchenort | Abschlußdatum der Recherche | Prüfer |
|---|---|---|
| BERLIN | 07-04-1989 | CORDERO ALVAREZ M. |

EPO FORM 1503 03.82 (P0403)

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

  .......................................................................
& : Mitglied der gleichen Patentfamilie, übereinstimmendes Dokument